# EUROPEAN PATENT SPECIFICATION

(11) **EP 2 682 109 B1**
(45) Date of publication and mention of the grant of the patent: **13.12.2017**
(21) Application number: 13175065.5
(22) Date of filing: 04.07.2013
(51) Int. Cl.: A61K 9/20, A61K 31/192, A61K 31/426

(54) **Combined immediate release formulations of flurbiprofen and famotidine**
Kombinierte Formulierungen mit sofortiger Freisetzung von Flurbiprofen und Famotidin
Formulations à libération immédiate combinée de flurbiprofène et famotidine

(30) Priority: 05.07.2012 TR 201207841
(43) Date of publication of application: 08.01.2014
(73) Proprietor: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- EP-A1- 2 260 837
- US-A1- 2005 163 847
- TAHA ALI S ET AL: "Famotidine for the prevention of gastric and duodenal ulcers caused by nonsteroidal antiinflammatory drugs", NEW ENGLAND JOURNAL OF MEDICINE, THE, MASSACHUSETTS MEDICAL SOCIETY, WALTHAM, MA, US, vol. 334, no. 22, 30 May 1996 (1996-05-30) , pages 1435-1439, XP002550689, ISSN: 0028-4793, DOI: 10.1056/NEJM199605303342204

## Description

### Technical Field:

The present invention is related to a pharmaceutical formulation which comprises Flurbiprofen as NSAID having antiinflamatory, analgesic and antipyretic effect together with Famotidine as an H2 receptor antagonist used for preventing and minimizing the gastrointestinal side effects of NSAID.

### Background of the Invention:

Flurbiprofen (Formula 1) is a propionic acid derivative, also known as NSAID (non-steroidal anti-inflammatory drug), having analgesic, anti-inflammatory and antipyretic activities.

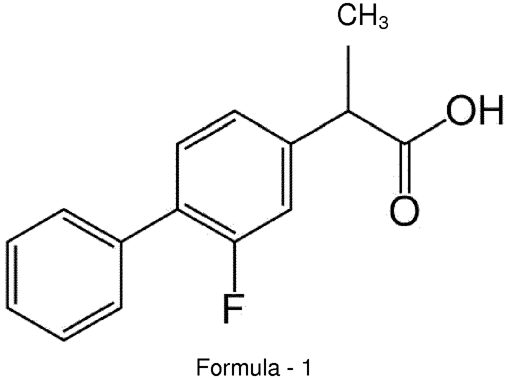

Flurbiprofen is used to alleviate the pain in musculoskeletal system and joint disorders such as ankylosing spondylitis, osteoarthritis and rheumatoid arthritis; in soft tissue injuries such as sprains and strains; and for posoperative pain, as well as pains including migraine headache and for painful and difficult menstruation.

At the same time, flurbiprofen is used as a pastil for the symptomatic healing of sore throat. Besides tablet and pastil, additionally gel, capsule and solution forms are also among the dosage forms of flurbiprofen.

The most commonly used flurbiprofen film tablet releases flurbiprofen immediately and it is immediately absorbed in such a way that its bioavailability reaches to 96%. The most important reason for formulating the flurbiprofen in this way is to have a molecule, which have antiinflamatory, analgesic and antipyretic effect, acting quickly. Therefore, the initiation of release when the flurbiprofen reaches to the gastrointestinal system is important in terms of drug's effect.

The absorption of the flurbiprofen mostly takes place in the gastrointestinal system, in the duodenal part of the stomach and the small intestine. Therefore, the initiation of the release when the flurbiprofen reaches the gastrointestinal system is important in terms of drug's effect.

Besides the wide usage area, the flurbiprofen also has a wide range of side effects. These side effects are mostly seen in the gastrointestinal system and the most frequent one affects the stomach mucosa. These side effects can be observed as gastric erosion, peptic ulcer, major upper gastrointestinal system bleedings, alterations in the intestine permeability and inflame mation.

These side effects are caused by the fact that flurbiprofen inhibits cyclooxygenase enzyme activity and prostaglandin synthesis, thereby damaging gastrointestinal mucosa due to increased gastric acid level. In fact, the mechanism of effect of flurbiprofen in the treatment is realized by the inhibition of prostaglandin and cyclooxygenase enzymes present in the tissues. However, when the same enzymes present also in the gastrointestinal mucosa cells are inhibited, the side effects listed above are observed.

In order to prevent or minimize the side effects emerged as a result of the increase in the acid level caused by NSAID molecules such as flurbiprofen, a lot of studies have been executed in prior art.

For example, in order to minimize gastric damage, flurbiprofen is enteric coated (Hashmat D., Shoaib H., Mehmood Z. (2008) AAPS PharmaSci Tech 9(1): 116-121) or formulated such that controlled release thereof will be performed (EP0234670).

Moreover, the researchers have combined NSAID molecules with acid inhibitors, in addition to formulation studies performed, in order to prevent or treat gastrointestinal damage. Acid level of gastric fluid is balanced, with acid inhibitors in this combination.

Patents numbered US5204118 and US5417980 of prior art combine NSAID molecules with proton pump inhibitors, so as to balance gastric acid. Since the researches on this combination have been carried out for many years, NSAID molecules have been combined with many molecules balancing gastric acid so far.

The most important ones among these molecules may be listed as proton pump inhibitors, H2 receptor antagonists and prostaglandin analogues. Among the H2 receptor antagonists, it is know that the molecule called famotidine (Formula 2) is used in the treatment of gastric and duodenal ulcer.

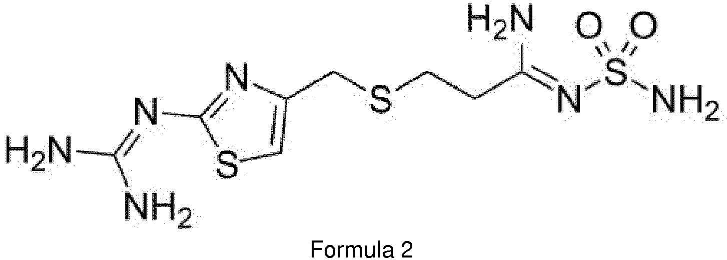

In order to effectively administer the studied NSAID - acid inhibitor combinations to the human body, various studies for multilayer tablet formulations have also been carried out. One of the primary reasons for this situation is to lessen or prevent incompatibility of the substances to be formulated with one another. Moreover, another important reason is that the formulations of two active substances with different release properties can be provided in the same form. The patent numbered EP1411900 relates to a multilayer tablet comprising NSAID and acid inhibitors. Here, NSAIDs are present in the inner core and released after the acid inhibitors present in the outer layer. US 2005/163847 and EP 2260837, both disclose multilayer tablets comprising flurbiprofen and famotidine. When a drug substance is combined with another drug substance in order to minimize or prevent the side effects of the drug substance, these two drug substances must be released, dissolved and absorbed harmoniously. For the efficiency of the formulation, an easy disintegration of both drug substances at a desired time is an important factor.

In order to minimize or prevent the side effects of flurbiprofen; it is very important for the molecule, in which the molecule is used in combination with the H2 receptor antogonists, to release both drug substances at the desired time and desired efficiency. Under normal circumstances, the release and consequently, the absorbtion of the formulations of prior art which are combined with H2 receptor antagonists to minimize the side effects of flurbiprofen which is released quickly, may be realized very late, as these formulations are formulated in a way to perform a delayed release. In this case, the bioavailability of the flurbiprofen is negatively affected.

Due to the intermolecular incompatibility in these formulations, the suitable formulation with which the desired effect will be actualized can not be obtained. Besides that, the dissolution profile of flurbiprofen whose release in gastrointestinal system, where it is expected to be released and absorbed, is not achieved will be negatively affected from this situation. Besides them, side effects of it will arise due to the increase in its impurity.

Eventually, there is still a need today for the pharmaceutical formulations in which deficient dissolution profile and bioavalibility as a result of delayed release are not observed and likewise, which are formulated in such a way that they will be released effectively with the H2 receptor antagonist while the side effects of flurbiprofen molecule, which is formulated for being used together with the H2 receptor antagonists, are diminished.

### Description of the Figures

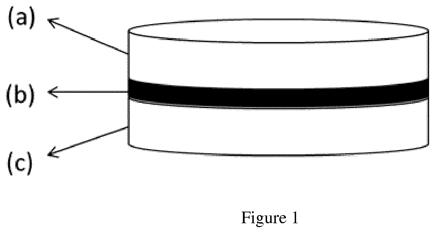

**Figure 1** shows an oral solid multilayer tablet formulation which comprises i) a first layer (a) comprising the first drug substance, ii) a second layer (c) comprising the second drug substance and iii) a barrier layer (b) separating these two layers.

### Detailed Description of the Invention

The present invention is related to a pharmaceutical formulation which comprises flurbiprofen having antiinflamatory, analgesic and antipyretic effect together with famotidine used for preventing and minimizing the gastrointestinal side effects of NSAID. Here, famotidine increases acid level of gastric fluid by inhibiting acid secretion, thereby preventing or minimizing such diseases as gastric erosion, peptic ulcer, major upper gastrointestinal system bleedings, alteration the intestine permeability and inflammation, all of which are caused by NSAID. The present invention relates to the fact that the pharmaceutical formulation comprising NSAID as first active substance and H2 receptor antagonist as second active substance is a novel solid oral dosage form and the solid oral dosage form is in the form of multilayer tablet.

The present invention is related to a multilayer tablet formulation according to claim 1 which comprises i) a
first layer comprising NSAID, ii) a second layer comprising the H2 receptor antagonist molecule and iii) a barrier layer separating these two molecules from each other. The present invention is related to the multilayer tablet formulation which comprises flurbiprofen as NSAID and famotidine as H2 receptor antagonist.

The present invention may comprise flurbiprofen and famotidine molecules in the multilayer tablet formulation thereof as pharmaceutically acceptable salts, enantiomers, racemates, polymorphs, esters and/or hydrates thereof.

The present invention is related to an oral solid multilayer tablet formulation comprising i) a first layer comprising flurbiprofen molecule as the first drug substance, ii) a second layer comprising famotidine as the second drug substance and iii) a barrier layer separating these two layers from each other, characterized in that; these 3 layers are lined up as a sandwich (Figure 1).

Here, the barrier layer is present in the middle in such a way that it seperates the flurbiprofen and famotidine layers and the flurbiprofen and famotidine layers are present at the outer part of the barrier layer. By the virtue of the barrier layer present in the middle part, interaction between the flurbiprofen and famotidine molecules is prevented. The present invention relates to formulating the solid oral multilayer tablet, which comprises flurbiprofen as the first drug substance and famotidine as the second drug substance, in a way to release both drug substances rapidly, wherein, this tablet comprises
i) an immediate release layer comprising flurbiprofen and pharmaceutically acceptable excipient,
ii) an immediate release layer comprising famotidine and pharmaceutically acceptable excipient, and
iii) a barrier layer provided between these two layers.

None of the layers of the multilayer tablet according to the present invention are formulated in such a way to alter the release profiles of flurbiprofen or famotidine molecules. Thus, the multilayer tablet according to the present invention has two layers comprising the flurbiprofen and famotidine molecules seperately and it is characterized in releasing these molecules together and rapidly.

Thus, as the release is initiated when the flurbiprofen reaches to the gastrointestinal system by way of the multilayer tablet, in which flurbiprofen and famotidine molecules can be released immediatly, its dissolution profile and bioavailability are not affected and the possible gastrointestinal side effects are rapidly minimized or prevented with the immediate release of famotidin.

Additionaly, as it is formulated according to the present invention, famotidin increases the pH of stomach as it inhibits the acid secreation, which is also the reason for its effect on gastrointestinal side effects when the famotidine molecule is released immediatly. Thus, with the increasing stomach pH, the flurbiprofen which initiates being released when it enters the stomach resolves better and its dissolution profile increases.

The multilayer tablet according to the present invention is formulated in such a way that it increases the pH of the stomach and the dissolution profile of the flurbiprofen consequently thanks to the release profile of famotidine.

According to the present invention, pharmaceutically acceptable excipients which are present in the immediate release layers comprising famotidine and flurbiprofen, can be selected from the group including, but not limited to, binding agents, disintegrants, glidants, lubricants, plasticizers, surface active agents, preservatives and mixtures thereof.

Suitable binders may include, but not limited to polymetacrylate, polyvinylpyrrolidone (povidon), hydroxypropyl methyl cellulose (HPMC), hydroxypropyl cellulose (HPC), carboxy methyl cellulose (CMC), methyl cellulose (MC), hydroxy ethyl cellulose, sodium carboxy methyl cellulose (NaCMC), carboxymethyl cellulose calcium, ethyl cellulose, polyethylene oxide, gelatin, starch, pregelatinized starch, xanthan gum, guar gum, alginate, carrageenan, pectin, carbomer, cellulose acetat phytalate, hydroxy propyl starch, polaxomer, poly ethylene glychol or mixtures thereof.

Suitable disintegrants of the invention, may include, but not limited to, microcrystalline cellulose, croscarmellose sodium, xylitol, polyplasdone (1-ethenylpyrrolidin-2-one), crospovidone, low-substituted hydroxypropyl cellulose (L-HPC) and sodium starch glycolate or mixtures thereof.
Suitable glidants of the invention may include but not limited to silicon dioxide, magnesium trisilicate, starch, talc, colloidal silicon dioxide or silicon hydrogel or mixtures thereof.

Suitable lubricants of the invention may include but not limited to magnesium stearate, sodium stearyl fumarate, polyethylene glycol, stearic acid, metal stearates, boric acid, sodium chloride benzoate and acetate, sodium or magnesium lauryl sulfate or mixtures thereof.

Suitable plasticizers of the invention may comprise but not limited to triethyl citrate, triacetin, citric acid esters, phthalic acid esters dibutyl sebacate, cetyl alcohol, polyethylene glycol, polysorbate or mixtures thereof.

Suitable surfactants of the invention, may include but not limited to dioctyl sulfosuccinate, polysorbates and polyoxyethylene alkyl esters and ethers thereof, glyceryl monolaurate saponins, sorbitan laurate, sodium lauryl sulfate, magnesium lauryl sulfate or mixtures thereof.

Suitable preservatives of the invention may include but not limited to methyl paraben, propyl paraben and salts thereof (e.g. sodium or potassium salts), sodium benzoate, citric acid, benzoic acid, butylated hydroxytoluene and butylated hydroxyanisole or mixtures thereof.

On the other hand, the immediate release layer according to the present invention which contains famotidin, comprises colloidal silicone dioxide as excipient. Thus, the uniformity of the famotidine molecule, which inhibits the acid secration and consequently icreases the pH of the stomach, is provided in the tablet and the release profile is improved when the tablet is dissolved.

The immediate release layer comprising famotidine according to the present invention comprises colloidal silicone dioxide and microcrystaline cellulose as excipiants.

According to the present invention, colloidal silicone dioxide in the immediate release layer comprising famotidine is present in an amount of about 0.1 to 10 % and preferably 0.1 to 2 % by weight of the layer.

According to the present invention, microcrystalline cellulose in the immediate release layer comprising famotidine is preferably microcrystalline cellulose pH 101 and it is present in an amount of about 60 to 80 % and preferably 65 to 75 % by weight of the layer.

In the immediate release layer containing famotidine according to the present invention, the weight ratio of microcrystalline cellulose excipient to colloidal silicone dioxide excipient is about 4 to 800 and preferably 25 to 200.

In the immediate release layer that is formulated together with these excipients, the uniformity of the drug substances and excipients increases. Thus, the immediate release layer disintegrates easier and the famoditine is released faster. The pH of the stomach increases with the immediate releasing famotidine. The dissolution profile of the flurbiprofen molecule with an increased dissolution rate, is improved thanks to the increasing pH.

As a result, with the multilayer tablet comprising flurbiprofen and famotidine drug substances which are present in the immediate release layers formulated according to the invention, an antiinflamatory, analgesic and antipyretic drug from, which have an increased dissolution profile and decreased side effects, is developed.

The multilayer tablet according to the invention comprises the flurbiprofen and famotidine drug substances with a ratio of about 7:1 and preferably 5:1 by weight. The low amount of famotidine in the tablet may cause the famotidine not to disperse homogenously in the formulation. This situation may cause a content uniformity problem and therefore, the non homogenous tablets do not release equal amount of famotidine drug substance when they are disintegrated. That is why the famotidine should disintegrate in an effective manner.

When the famotidine is applied in high dosages; it may cause minor side effects such as headache, weakness, fatigue, fever; and especially in patients with kidney failure, it may also cause side effects related with the central nervous system such as anxiety, depression and mental disorders. The multilayer tablet formulation according to the invention comprises famotidine in an amount of about 10 to 50 mg and preferably 15 to 35 mg.

The immediate release layer according to the present invention should release equal amounts of famotidin. Therefore, this immediate release layer must be formulated in such a way that it must realize the release of famotidine, which is preferably 5 unit less than flurbiprofen, immediately and effectively.

In order to obtain this immediate formulation, microcrystalline cellulose and colloidal silicone dioxide which facilitate the disintegration of the formulation should be present in the formulation as pharmaceutically acceptable excipients. Here, the weight ratio of microcrystalline cellulose to colloidal silicone dioxide is formulated in accordance with the invention and it is about 4-800 and preferably 25-200.

Thus, the immediate release layer containing low amount of famotidine disintegrates efficiently, the positive effect of famotidine on the pH of stomach is actualized rapidly and the dissolution profile and bioavailibility of flurbiprofen improve with the increasing pH.

The present invention comprises a first layer that comprises flurbiprofen, a second layer that comprises famotidin and a barrier layer provided between these two layers and it is characterized in that, none of these layers comprise a coating. In order to keep the drug substance and the excipients stable, colloidal silicone dioxide and microcrystalline cellulose are provided in the flurbiprofen and famotidine layers which do not contain coating.

The multilayer tablet according to the present invention comprises famotidine in an amount of 5-25 % by weight and preferably 10-15 % by weight, in the immediate release layer which comprises famotidine and pharmaceutically acceptable excipient.

The multilayer tablet according to the present invention comprises flurbiprofen in an amount of 20-50 % by weight and preferably 30-40 % by weight, in the immediate release layer which comprises famotidine and pharmaceutically acceptable excipient.

The present invention is related to a multilayer tablet that has an immediate release layer comprising famotidine and it comprises;
a) about 5 to 25% by weight of famotidine,
b) about 10 to 20 % by weight of pregelatinized starch,
c) about 60 to 80 % by weight of microcrystalline cellulose,
d) about 0.1 to 10 % by weight of colloidal silicon dioxide,
e) about 0.1 to 10 % by weight of magnesium stearate.

The present invention is related to a multilayer tablet that has an immediate release layer comprising flurbiprofen, which comprises;
a) about 20 to 50% by weight of flurbiprofen,
b) about 5 to 60% by weight of lactose,
c) about 5 to 30 % by weight of microcrystalline cellulose,
d) about 0.5 to 10 % by weight of crosscarmellose sodium,
e) about 2 to 10 % by weight of hydroxypropyl cellulose,
f) about 0.1 to 10 % by weight of colloidal silicon dioxide,
g) about 0.1 to 10 % by weight of magnesium stearate.

In another aspect, the present invention is related to a sandwich shaped multilayer tablet which comprises a barrier layer that seperates the first immediate release layer comprising flurbiprofen and the second immediate release layer comprising famotidine and characterized in that the barrier layer forms the middle layer of the sandwich shaped multilayer tablet and contains pharmaceutically acceptable excipients. By means of this barrier layer, the incompatibility of flurbiprofen and famotidine molecules within the formulation is minimized or prevented.

As a pharmaceutically acceptable excipient, the barrier layer according to the present invention comprises binding agents, dispersants, glidants, lubricants, plasticizers, surface active agents, preservatives or mixtures thereof, it especially comprises;
a) about 0.1 to 10 % by weight of hydroxypropyl cellulose,
b) about 70 to 99 % by weight of microcrystalline cellulose,
c) about 0.1 to 10 % by weight of yellow iron oxide,
d) about 0.1 to 10 % by weight of colloidal silicon dioxide,
e) about 0.1 to 10 % by weight of magnesium stearate.

The multilayer tablet according to the present invention is composed of flurbiprofen immediate release layer, famotidine immediate release layer and barrier layer and in each layer it comprises colloidal silicone dioxide and microcrystaline cellulose as pharmaceutically acceptable excipiant.

In its each layer, the multilayer tablet according to the present invention comprises colloidal silicone dioxide as excipient, wherein the amount of colloidal silicone dioxide by weight is about 0.1 to 5 mg.

In its each layer, the multilayer tablet according to the present invention comprises microcrystalline cellulose as excipient, wherein the amount of microcrystalline cellulose in each layer by weight is about 50 to 150 mg.

The ratio of microcrystalline cellulose and colloidal silicone dioxide present as excipients in each layer of the multilayer tablet according to the present invention is between about 4-800 and preferably 25-200 by weight. Another aspect is a method for preparing the said multilayer tablet which comprises the following steps:
a) for the preparation of the first layer;
   i. flurbiprofen, microcrytaline cellulose, lactose monohydrate and a part of hydroxypropyl cellulose are transferred to the fluid bed dryer,
   ii. an aqueous solution is prepared with the remaining part of the hydroxypropyl cellulose, granulated, dried and grinded in the fluid bed dryer and transferred to the container,
   iii. croscarmellose sodium and colloidal silicone dioxide are added to the same container,
   iv. the powder mixture in this container is blended,
   v. magnesium stearate is added to the mixture obtained in step iii) and blended,
b) for the preparation of the second layer;
   i. famotidine, pregelatinized starch and microcrystalline cellulose are picked,
   ii. the mixture obtained in step i) is granulated with water-alcohol mixture, grinded and dried in oven,
   iii. the dried mixture is grinded again and the colloidal silicone dioxide is put to the same container by being sieved,
   iv. the powder mixture in this container is blended,
   v. magnesium stearate is added to the mixture obtained in step iii) and blended,
c) for the preparation of the barrier layer;
   i. a part of microcrystalline cellulose and hydroxypropyl cellulose are transferred to the container,
   ii. remaining part of microcrystalline cellulose, iron oxide yellow and colloidal silicone dioxide are sieved and transferred to the same container,
   iii. the powdered mixture in this container is blended,
   iv. magnesium stearate is added to the mixture obtained in step iii) and blended,
d) the homogeneous mixtures obtained in step a), b) ve c) are compressed to obtain a sandwich shaped tablet.

### Examples:

In the present invention, the following examples are defined as the preferred compositions of the present invention. Although the examples are not intended to limit the scope of the present invention, it should be considered in the light of the description detailed above.

### Example 1

The present example is directed to a sandwich shaped trilayer tablet comprising flurbiprofen and famotidine. The above formulation is prepared by obtaining separately three layers and by compressing them to obtain a sandwich shaped tablet. The preparation process of the tablet is detailed in the description.

| | **Ingredient** | **Amount (mg)** |
|---|---|---|
| **First Layer** | Flurbiprofen | 100 |
| | Lactose monohydrate | 124.5 |
| | Microcrystalline cellulose | 55 |
| | Croscarmellose sodium | 7.3 |
| | Hydroxypropyl cellulose | 9.8 |
| | Colloidal silicon dioxide | 2.1 |
| | Magnesium Stearate | 1.3 |
| | **Total** | **300** |
| **Barrier Layer** | Hydroxypropyl cellulose | 8 |
| | Microcrystalline cellulose | 139.1 |
| | Yellow iron oxide | 0.6 |
| | Colloidal silicon dioxide | 0.8 |
| | Magnesium Stearate | 1.5 |
| | **Total** | **150** |
| **Second Layer** | Famotidine | 20 |
| | Microcrystalline cellulose | 105.5 |
| | Croscarmellose sodium | 7.5 |
| | Hydroxypropyl cellulose | 15 |
| | Colloidal silicon dioxide | 1 |
| | Magnesium Stearate | 1 |
| | **Total** | **150** |

### Example 2

As example 1, the above example is also directed to a sandwich shaped trilayer tablet comprising flurbiprofen and famotidine.

| | **Ingredient** | **Amount (mg)** |
|---|---|---|
| **First Layer** | Flurbiprofen | 100 |
| | Lactose monohydrate | 124.5 |
| | Microcrystalline cellulose | 55 |
| | Croscarmellose sodium | 7.3 |
| | Hydroxypropyl cellulose | 9.8 |
| | Colloidal silicon dioxide | 2.1 |
| | Magnesium Stearate | 1.3 |
| | **Total** | **300** |
| **Barrier Layer** | Hydroxypropyl cellulose | 8 |
| | Microcrystalline cellulose | 139.1 |
| | Yellow iron oxide | 0.6 |
| | Colloidal silicon dioxide | 0.8 |
| | Magnesium Stearate | 1.5 |
| | **Total** | **150** |
| **Second Layer** | Famotidine | 20 |
| | Microcrystalline cellulose | 105.5 |
| | Pregelatinized starch | 23 |
| | Colloidal silicon dioxide | 1 |
| | Magnesium Stearate | 1 |
| | **Total** | **150** |

### Example 3

The following Table 1 is about dissolution profile of flurbiprofen depending on the medium pH. This table is obtained by tests which are carried out by USP2 Paddle Dissolution Method. As the pH increases, flurbiprofen has a greater dissolution profile. Table 1 shows also that flurbiprofen is immediately released from the trilayer tablet of the present invention.

**Table 1 - Dissolution profile of flurbiprofen depending on medium pH**

| | **0.1 N HCI, 900 mL, 50 rpm** | **pH=4.5 sodium acetate buffer, 900 mL, 50 rpm** | **pH=7.2, 0.05 M KH2PO4 buffer, 900 mL, 50 rpm** |
|---|---|---|---|
| **Time** | **Avg**.(%) | **Avg**.(%) | **Avg**. **(%)** |
| **0** | 0 | 0 | 0 |
| **5** | 8 | 20 | 75 |
| **10** | 10 | 28 | 91 |
| **15** | 10 | 33 | 96 |
| **20** | 11 | 35 | 97 |
| **30** | 11 | 39 | 98 |
| **45** | 11 | 40 | 99 |
| **60** | 11 | 40 | 100 |

## Claims

1. A solid oral multilayer tablet comprising
i) a first immediate release layer (a) comprising flurbiprofen as drug substance and pharmaceutically acceptable excipient,
ii) a second immediate release layer (c) comprising famotidine as drug substance and pharmaceutically acceptable excipient, and
iii) a barrier layer (b) provided between these two layers, comprising pharmaceutically acceptable excipient,
wherein these 3 layers are lined up in sandwich form, in such a way that the barrier layer is provided in the middle, and the first and the second layers comprising the drug substances are formulated in a way to release the drug substances immediately.

2. The solid oral multilayer tablet according to claim 1 wherein, the first layer (a) comprises 20 to 50% by weight of the layer flurbiprofen as drug substance.

3. The solid oral multilayer tablet according to claim 1 wherein, the second layer (c) comprises 5 to 25% by weight of the layer famotidine as drug substance.

4. The solid oral multilayer tablet according to claim 1 wherein, the pharmaceutically acceptable excipient in its content comprises at least one of the substances selected among binding agents, disintegrants, glidants, lubricants, plasticizers, surfactants, preservatives or mixtures thereof.

5. The solid oral multilayer tablet according to claim 4, wherein the binders in its content are selected among polymetacrylate, polyvinylpyrrolidone (povidon), hydroxypropyl methyl cellulose (HPMC), hydroxypropyl cellulose (HPC), carboxy methyl cellulose (CMC), methyl cellulose (MC), hydroxy ethyl cellulose, sodium carboxy methyl cellulose (NaCMC), carboxymethyl cellulose calcium, ethyl cellulose, polyethylene oxide, gelatin, starch, xanthan gum, guar gum, alginate, carrageenan, pectin, carbomer, cellulose acetat phytalate, hydroxy propyl starch, poloxamer, poly ethylene glycol or mixtures thereof.

6. The solid oral multilayer tablet according to claim 4, wherein the disintegrants in its content are selected among microcrystalline cellulose, croscarmellose sodium, xylitol, polyplasdone (1-ethenylpyrrolidin-2-one), crospovidone, low-substituted hydroxypropyl cellulose (L-HPC) and sodium starch glycolate or mixtures thereof.

7. The solid oral multilayer tablet according to claim 4 wherein, the glidants in its content are selected among silicon dioxide, magnesium trisilicate, starch, talc, colloidal silicon dioxide or silicon hydrogel or mixtures thereof.

8. The solid oral multilayer tablet according to any of the previous claims wherein the second layer (c) which comprises famotidine as drug substance and pharmaceutically acceptable excipient comprises colloidal silicone dioxide and microcrystalline cellulose as excipient.

9. The solid oral multilayer tablet according to claim 8 wherein the colloidal silicone dioxide in the immediate release layer (c) comprising famotidine is present in an amount of 0.1 to 10 % by weight of the layer.

10. The solid oral multilayer tablet according to claim 9 wherein the colloidal silicone dioxide in the immediate release layer (c) comprising famotidine is present in an amount of 0.1 to 2 % by weight of the layer.

11. The solid oral multilayer tablet according to claim 8 wherein the microcrystalline cellulose in the immediate release layer (c) comprising famotidine is present in an amount of 60 to 80 % by weight of the layer.

12. The solid oral multilayer tablet according to claim 11 wherein the microcrystalline cellulose in the immediate release layer (c) comprising famotidine is present in an amount of 65 to 75 % by weight of the layer.

13. The solid oral multilayer tablet according to claim 8 wherein the weight ratio of excipient microcrystalline cellulose to excipient colloidal silicone dioxide in the immediate release layer (c) comprising famotidine is between 4 and 800.

14. The solid oral multilayer tablet according to claim 1 wherein the tablet comprises;
a. an immediate release layer (a) comprising flurbiprofen as drug substance and
i. 5 to 60% by weight of lactose,
ii. 5 to 30 % by weight of microcrystalline cellulose,
iii. 0.5 to 10 % by weight of crosscarmellose sodium,
iv. 2 to 10 % by weight of hydroxypropyl cellulose,
v. 0.1 to 10 % by weight of colloidal silicon dioxide,
vi. 0.1 to 10 % by weight of magnesium stearate,
as excipient,
b. an immediate release layer (c) comprising famotidine as drug substance and
i. 10 to 20 % by weight of pregelatinized starch,
ii. 60 to 80 % by weight of microcrystalline cellulose,
iii. 0.1 to 10 % by weight of colloidal silicon dioxide,
iv. 0.1 to 10 % by weight of magnesium stearate,
as excipient,
c. a barrier layer (b) provided between these two layers and comprising
i. 0.1 to 10 % by weight of hydroxypropyl cellulose,
ii. 70 to 99 % by weight of microcrystalline cellulose,
iii. 0.1 to 10 % by weight of yellow iron oxide,
iv. 0.1 to 10 % by weight of colloidal silicon dioxide,
v. 0.1 to 10 % by weight of magnesium stearate.
as excipient.

## Patentansprüche

1. Eine feste orale Mehrschichttablette, umfassend
(i) eine erste Schicht mit sofortiger Freisetzung (a), umfassend Flurbiprofen als Arzneistoff und einen pharmazeutisch verträglichen Exzipienten,
(ii) eine zweite Schicht mit sofortiger Freisetzung (c), umfassend Famotidin als Arzneistoff und einen pharmazeutisch verträglichen Exzipienten, und
(iii) eine Barriereschicht (b), vorgesehen zwischen diesen zwei Schichten, umfassend einen pharmazeutisch verträglichen Exzipienten,
wobei diese 3 Schichten so in Sandwichform ausgerichtet sind, dass die Barriereschicht in der Mitte vorgesehen ist, und die erste und die zweite Schicht, die die Arzneistoffe umfassen, in einer Weise formuliert sind, dass die Arzneistoffe sofort freigesetzt werden,

2. Feste orale Mehrschichttablette nach Anspruch 1, wobei die erste Schicht (a) zu 20 bis 50 Gew.-% der Schicht Flurbiprofen als Arzneistoff umfasst.

3. Feste orale Mehrschichttablette nach Anspruch 1, wobei die zweite Schicht (c) zu 5 bis 25 Gew.-% der Schicht Famotidin als Arzneistoff umfasst.

4. Feste orale Mehrschichttablette nach Anspruch 1, wobei der pharmazeutisch verträgliche Exzipient in ihrem Inhalt mindestens einen der Stoffe, ausgewählt unter Bindemitteln, Sprengmitteln, Fließregulierungsmitteln, Gleitmitteln, Weichmachern, grenzflächenaktiven Mitteln, Konservierungsmitteln oder Gemischen davon, umfasst.

5. Feste orale Mehrschichttablette nach Anspruch 4, wobei die Bindemittel in ihrem Inhalt ausgewählt sind unter Polymethacrylat, Polyvinylpyrrolidon (Povidon), Hydroxypropylmethylcellulose (HPMC), Hydroxypropylcellulose (HPC), Carboxymethylcellulose (CMC), Methylcellulose (MC), Hydroxyethylcellulose, Natriumcarboxymethylcellulose (NaCMC), Calciumcarboxymethylcellulose, Ethylcellulose, Polyethylenoxid, Gelatine, Stärke, Xanthan, Guarkernmehl, Alginat, Carrageenan, Pektin, Carbomer, Celluloseacetatphthalat, Hydroxypropylstärke, Poloxamer, Polyethylenglykol oder Gemischen davon.

6. Feste orale Mehrschichttablette nach Anspruch 4, wobei die Sprengmittel in ihrem Inhalt ausgewählt sind unter mikrokristalliner Cellulose, Croscarmellose-Natrium, Xylitol, Polyplasdone (1-Ethenylpyrrolidin-2-on), Crospovidon, niedrig substituierter Hydroxypropylcellulose (L-HPC) und Natriumstärkeglykolat oder Gemischen davon.

7. Feste orale Mehrschichttablette nach Anspruch 4, wobei die Fließregulierungsmittel in ihrem Inhalt ausgewählt sind unter Siliciumdioxid, Magnesiumtrisilicat, Stärke, Talkum, kolloidalem Siliciumdioxid oder Siliciumhydrogel oder Gemischen davon.

8. Feste orale Mehrschichttablette nach einem der vorangehenden Ansprüche, wobei die zweite Schicht (c), die Famotidin als Arzneistoff und einen pharmazeutisch verträglichen Exzipienten umfasst, kolloidales Siliciumdioxid und mikrokristalline Cellulose als Exzipient umfasst.

9. Feste orale Mehrschichttablette nach Anspruch 8, wobei das kolloidale Siliciumdioxid in der Famotidin umfassenden Schicht mit sofortiger Freisetzung (c) in einer Menge von 0,1 bis 10 Gew.-% der Schicht vorliegt.

10. Feste orale Mehrschichttablette nach Anspruch 9, wobei das kolloidale Siliciumdioxid in der Famotidin umfassenden Schicht mit sofortiger Freisetzung (c) in einer Menge von 0,1 bis 2 Gew.-% der Schicht vorliegt.

11. Feste orale Mehrschichttablette nach Anspruch 8, wobei die mikrokristalline Cellulose in der Famotidin umfassenden Schicht mit sofortiger Freisetzung (c) in einer Menge von 60 bis 80 Gew.-% der Schicht vorliegt.

12. Feste orale Mehrschichttablette nach Anspruch 11, wobei die mikrokristalline Cellulose in der Famotidin umfassenden Schicht mit sofortiger Freisetzung (c) in einer Menge von 65 bis 75 Gew.-% der Schicht vorliegt.

13. Feste orale Mehrschichttablette nach Anspruch 8, wobei das Gewichtsverhältnis von Exzipient mikrokristalline Cellulose zu Exzipient kolloidales Siliciumdioxid in der Famotidin umfassenden Schicht mit sofortiger Freisetzung (c) zwischen 4 und 800 beträgt.

14. Feste orale Mehrschichttablette nach Anspruch 1, wobei die Tablette umfasst:
a. eine Schicht mit sofortiger Freisetzung (a), umfassend Flurbiprofen als Arzneistoff und
i. 5 bis 60 Gew.-% Lactose,
ii. 5 bis 30 Gew.-% mikrokristalline Cellulose,
iii. 0,5 bis 10 Gew.-% Croscarmellose-Natrium,
iv. 2 bis 10 Gew.-% Hydroxypropylcellulose,
v. 0,1 bis 10 Gew.-% kolloidales Siliciumdioxid,
vi. 0,1 bis 10 Gew.-% Magnesiumstearat,
als Exzipient,
b. eine Schicht mit sofortiger Freisetzung (c), umfassend Famotidin als Arzneistoff und
i. 10 bis 20 Gew.-% vorverkleisterte Stärke,
ii. 60 bis 80 Gew.-% mikrokristalline Cellulose,
iii. 0,1 bis 10 Gew.-% kolloidales Siliciumdioxid,
iv. 0,1 bis 10 Gew.-% Magnesiumstearat,
als Exzipient,
c. eine Barriereschicht (b), vorgesehen zwischen diesen zwei Schichten und umfassend
i. 0,1 bis 10 Gew.-% Hydroxypropylcellulose,
ii. 70 bis 99 Gew.-% mikrokristalline Cellulose,
iii. 0,1 bis 10 Gew.-% gelbes Eisenoxid,
iv. 0,1 bis 10 Gew.-% kolloidales Siliciumdioxid,
v. 0,1 bis 10 Gew.-% Magnesiumstearat,
als Exzipient.

## Revendications

1. Comprimé multicouches oral solide comprenant :
i) une première couche à libération immédiate (a) comprenant du flurbiprofène comme substance médicamenteuse et un excipient pharmaceutiquement acceptable ;
ii) une seconde couche à libération immédiate (c) comprenant de la famotidine comme substance médicamenteuse et un excipient pharmaceutiquement acceptable ; et
iii) une couche barrière (b) disposée entre ces deux couches, comprenant un excipient pharmaceutiquement acceptable,
dans lequel ces 3 couches sont rangées sous forme de sandwich, d'une manière telle que la couche barrière est disposée au milieu, et les première et seconde couches comprenant les substances médicamenteuses sont formulées de façon à libérer les substance médicamenteuses immédiatement.

2. Comprimé multicouches oral solide selon la revendication 1, dans lequel la première couche (a) comprend 20 à 50 % en poids de la couche, de flurbiprofène comme substance médicamenteuse.

3. Comprimé multicouches oral solide selon la revendication 1, dans lequel la seconde couche (c) comprend 5 à 25 % en poids de la couche, de famotidine comme substance médicamenteuse.

4. Comprimé multicouches oral solide selon la revendication 1, dans lequel l'excipient pharmaceutiquement acceptable dans son contenu comprend au moins l'une des substances choisies parmi les agents de liaison, les désintégrants, les agents de glissement, les lubrifiants, les plastifiants, les agents tensio-actifs, les conservateurs ou les mélanges de ceux-ci.

5. Comprimé multicouches oral solide selon la revendication 4, dans lequel les liants dans son contenu sont choisis parmi le polyméthacrylate, la polyvinylpyrrolidone (povidone), l'hydroxypropyl méthyl cellulose (HPMC), l'hydroxypropyl cellulose (HPC), la carboxy méthyl cellulose (CMC), la méthyl cellulose (MC), l'hydroxy éthyl cellulose, la carboxy méthyl cellulose sodique (NaCMC), la carboxy méthyl cellulose calcique, l'éthyl cellulose, le poly(oxyde d'éthylène), la gélatine, l'amidon, la gomme de xanthane, la gomme de guar, l'alginate, le carraghénane, la pectine, un carbomère, l'acétate phtalate de cellulose, l'hydroxy propyl amidon, un poloxamère, le polyéthylène glycol ou les mélanges de ceux-ci.

6. Comprimé multicouches oral solide selon la revendication 4, dans lequel les désintégrants dans son contenu sont choisis parmi la cellulose microcristalline, la croscarmellose sodique, le xylitol, la polyplasdone (1-éthénylpyrrolidin-2-one), la crospovidone, l'hydroxypropyl cellulose faiblement substituée (L-HPC) et le glycolate d'amidon sodique ou les mélanges de ceux-ci.

7. Comprimé multicouches oral solide selon la revendication 4, dans lequel les agents de glissement dans son contenu sont choisis parmi le dioxyde de silicium, le trisilicate de magnésium, l'amidon, le talc, le dioxyde silicium colloïdal ou l'hydrogel de silicium ou les mélanges de ceux-ci.

8. Comprimé multicouches oral solide selon l'une quelconque des revendications précédentes, dans lequel la seconde couche (c) qui comprend de la famotidine comme substance médicamenteuse et un excipient pharmaceutiquement acceptable comprend du dioxyde de silicium colloïdal et de la cellulose microcristalline comme excipient.

9. Comprimé multicouches oral solide selon la revendication 8, dans lequel le dioxyde de silicium colloïdal dans la couche à libération immédiate (c) comprenant de la famotidine est présent dans une quantité de 0,1 à 10 % en poids de la couche.

10. Comprimé multicouches oral solide selon la revendication 9, dans lequel le dioxyde de silicium colloïdal dans la couche à libération immédiate (c) comprenant de la famotidine est présent dans une quantité de 0,1 à 2 % en poids de la couche.

11. Comprimé multicouches oral solide selon la revendication 8, dans lequel la cellulose microcristalline dans la couche à libération immédiate (c) comprenant de la famotidine est présente dans une quantité de 60 à 80 % en poids de la couche.

12. Comprimé multicouches oral solide selon la revendication 11, dans lequel la cellulose microcristalline dans la couche à libération immédiate (c) comprenant de la famotidine est présente dans une quantité de 65 à 75 % en poids de la couche.

13. Comprimé multicouches oral solide selon la revendication 8, dans lequel le rapport en poids de l'excipient cellulose microcristalline à l'excipient dioxyde de silicium colloïdal dans la couche à libération immédiate (c) comprenant de la famotidine se situe entre 4 et 800.

14. Comprimé multicouches oral solide selon la revendication 1, dans lequel le comprimé comprend :
a. une couche à libération immédiate (a) comprenant du flurbiprofène comme substance médicamenteuse et
i. 5 à 60 % en poids de lactose ;
ii. 5 à 30 % en poids de cellulose microcristalline ;
iii. 0,5 à 10 % en poids de croscarmellose sodique ;
iv. 2 à 10 % en poids d'hydroxypropyl cellulose ;
v. 0,1 à 10 % en poids de dioxyde de silicium colloïdal;
vi. 0,1 à 10 % en poids de stéarate de magnésium,
comme excipient,
b. une couche à libération immédiate (c) comprenant de la famotidine comme substance médicamenteuse et
i. 10 à 20 % en poids d'amidon prégélatinisé ;
ii. 60 à 80 % en poids de cellulose microcristalline ;
iii. 0,1 à 10 % en poids de dioxyde de silicium colloïdal;
iv. 0,1 à 10 % en poids de stéarate de magnésium,
comme excipient,
c. une couche barrière (b) disposée entre ces deux couches et comprenant
i. 0,1 à 10 % en poids d'hydroxypropyl cellulose ;
ii. 70 à 99 % en poids de cellulose microcristalline ;
iii. 0,1 à 10 % en poids d'oxyde de fer jaune ;
iv. 0,1 à 10 % en poids de dioxyde de silicium colloïdal;
v. 0,1 à 10 % en poids de stéarate de magnésium,
comme excipient.
